**Europäisches Patentamt**

**European Patent Office**

**Office européen des brevets**

(19)

(11) Veröffentlichungsnummer: **0 270 048 B1**

(12) **EUROPÄISCHE PATENTSCHRIFT**

(45) Veröffentlichungstag der Patentschrift: **14.08.91**  (51) Int. Cl.⁵: **A61F 5/48**

(21) Anmeldenummer: **87117646.7**

(22) Anmeldetag: **28.11.87**

(54) **Vorrichtung zur Erzeugung eines Warnsignals bei unkontrolliertem Harnaustritt.**

(30) Priorität: **29.11.86 DE 3640900**

(43) Veröffentlichungstag der Anmeldung:
**08.06.88 Patentblatt  88/23**

(45) Bekanntmachung des Hinweises auf die
Patenterteilung:
**14.08.91 Patentblatt  91/33**

(84) Benannte Vertragsstaaten:
**AT CH DE IT LI NL SE**

(56) Entgegenhaltungen:
**CH-A- 532 395        DE-A- 1 566 377**
**DE-A- 2 948 768      US-A- 3 696 357**
**US-A- 3 809 078      US-A- 4 539 559**

(73) Patentinhaber: **Stegat, Harry, Prof. Dr.,**
**Waldwinkel 7**
**W-4400 Münster(DE)**

Patentinhaber: **Stegat geb. Heckmanns, Erika**
**Waldwinkel 7**
**W-4400 Münster(DE)**

(72) Erfinder: **Stegat, Harry, Prof. Dr.**
**Waldwinkel 7**
**W-4400 Münster(DE)**

(74) Vertreter: **Schulze Horn, Stefan, Dipl.-Ing.**
**M.Sc.**
**Goldstrasse 36**
**W-4400 Münster(DE)**

## Beschreibung

Die Erfindung betrifft eine Vorrichtung zur Erzeugung eines Warnsignals bei unkontrolliertem Harnaustritt, insbesondere zur Behandlung der Enuresis, mit einem im Bereich der Harnröhrenöffnung anzuordnenden Feuchtigkeitsfühlers, der auf einem Träger angeordnet ist, wobei der Feuchtigkeitsfühler mit durch eventuell austretenden Urin überbrückbaren unisolierten elektrischen Leiterbahnpaar versehen ist, mit einer mit dem Fühler elektrisch verbindbaren, am Körper tragbaren elektrischen oder elektronischen Schalteinheit zur Feststellung eines Auftretens vnn Feuchtigkeit am Fühler, wobei die Schalteinheit mittels eines Magneten über ein einen Teil der Schalteinheit bildendes Reedrelais schaltbar ist, und mit einem von der Schalteinheit im Ansprechfall ansteuerbaren Schallsignalgeber, und wobei der Fühler von einer leicht auswechselbaren, für eine einmalige Verwendung vorgesehenen, flüssigkeitsdurchlässigen Hülle umgeben ist. Eine derartige Vorrichting ist aus der DE-A-2 948 768 bekannt.

Aus der DE-A-2 948 768 ist ferner ein Vorwarngerät gegen Bettnässen bekannt, das eine Schutzhose mit einem Kontaktgeber aufweist, über den schon geringste Urinmengen akustisch angezeigt werden. Der Verschluß der Schutzhose ist mit einem Magneten und einem Reedschalter versehen, die durch Öffnen oder Verschließen der Hose den Kontakt zum Schallsignalgeber schließen oder unterbrechen.

Nachteilig ist hierbei aber, daß der Träger des Gerätes durch eine unbewußte Handlung eine Unterbrechung des Kontaktes vornehmen kann und ein Lerneffekt daher ausbleiben kann. Im Gegensatz dazu soll erfindungsgemäß die Aufgabe gelöst werden, daß eine bewußte Handlung ausgeführt werden muß und ein positiver Lerneffekt auftritt. Die Lösung dieser Aufgabe gelingt erfindungsgemäß durch eine Vorrichtung der eingangs genannten Art, bei welcher die Schalteinheit und damit der Schallsignalgeber nach einem Ansprechen durch Schließen des Reedrelais mit Hilfe eines Magneten abschaltbar ist.

Nach einem Ansprechen der Vorrichtung muß diese bzw. die zugehörige Schalteinheit von der sie tragenden Person durch einen manuellen Eingriff zurückgesetzt werden.

Es stellt sich weiterhin die Aufgabe, eine Vorrichtung der eingangs genannten Art zu schaffen, die die aufgeführten Nachteile vermeidet und die insbesondere funktionssicher ist, hohe hygienische Ansprüche erfüllt, einfach anwendbar und bedienbar ist und die kostengünstig herstellbar und betreibbar ist.

Die Vorrichtung ist weiterhin bevorzugt zum besonders zweckmäßigen Gebrauch derart ausgebildet, daß die Schalteinheit einen als Hybridschaltung ausgebildeten Schaltkreis mit zugeordneter Batterie aufweist, daß der Schaltkreis, die Batterie, das Reedrelais und der Schallsignalgeber gemeinsam innerhalb eines Gehäuses angeordnet sind und daß das Gehäuse seinerseits mit einem im Anwendungsfall über die Schulter eines Patienten führbaren Traggurt im Schulterbereich, d.h. in Ohrnähe des Patienten, verbindbar ist.

Im folgenden werden bevorzugte Ausführungsbeispiele der Erfindung anhand einer Zeichnung näher erläutert. Die Figuren der Zeichnung zeigen im einzelnen:

Figur 1    eine Vorrichtung gemäß Erfindung mit einem Fühler in einer ersten Ausführungsform in Ansicht,

Figur 2    einen Fühler mit Hülle als Teil der Vorrichtung, ebenfalls in Ansicht,

Figur 3    einen Fühler in einer zweiten Ausführungsform, ebenfalls in Ansicht und

Figur 4    eine Schalteinheit als Teil der Vorrichtung im Längsschnitt in prinzipieller Darstellung.

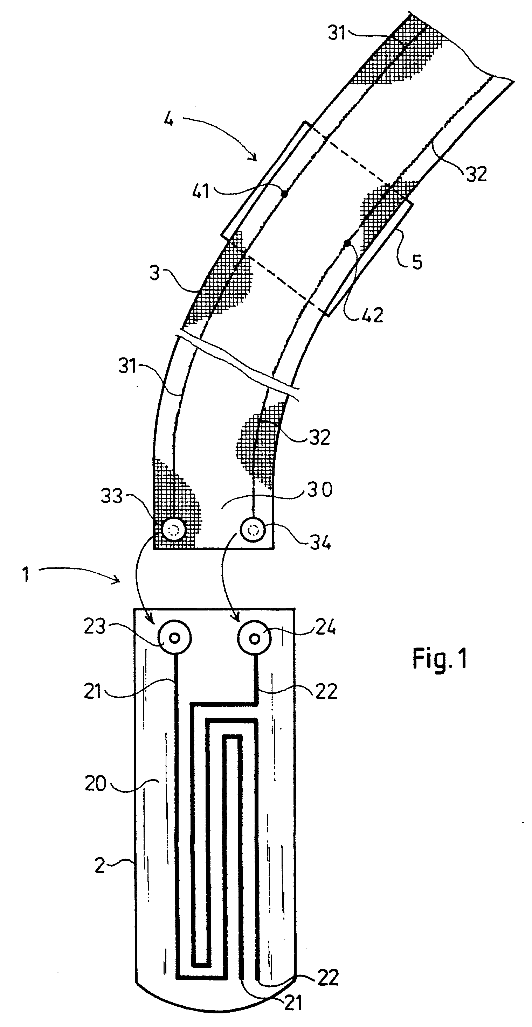

Wie die Figur 1 der Zeichnung zeigt, besteht das dargestellte Ausführungsbeispiel der Vorrichtung 1 im wesentlichen aus einem Feuchtigkeitsfühler 2, einem mit dem Fühler 2 zu verbindenden Traggurt 3 und einer elektrischen bzw. elektronischen Schalteinheit 4, welche an dem Gurt 3 befestigt ist.

Der Fühler 2 besteht aus einem Träger 20 aus einem flexiblen Kunststoff oder einem feuchtigkeitsabweisend beschichteten oder imprägnierten Papier und besitzt die Form eines länglichen Streifens. Einseitig ist auf einer der Flachseiten des Trägers 20 ein Leiterbahnpaar aus zwei Leiterbahnen 21 und 22 aufgebracht. Hierbei sind die Leiterbahnen vorzugsweise auf den Träger 20 aufgedruckt. Die beiden Leiterbahnen 21 und 22 enden offen, d. h. sie sind nicht elektrisch leitend miteinander verbunden, und verlaufen unter Überdeckung eines großen Teils der Oberfläche des Trägers 20 parallel zueinander. Die Funktionsweise des Fühlers 2 beruht darauf, daß bei einem Auftreffen von Feuchtigkeit auf dem Träger 20 eine elektrisch leitende Verbindung zwischen der Leiterbahn 21 und der Leiterbahn 22 gebildet wird. Diese elektrische Verbindung zwischen den beiden Leiterbahnen 21 und 22 wird von der Schalteinheit 4 erkannt und hat die Abgabe eines Warnsignals zur Folge. Eine derartige elektrische oder elektronische Schaltung ist an sich bekannt und braucht daher an dieser Stelle nicht erläutert zu werden.

Die elektrische Verbindung zwischen dem Fühler 2 und der Schalteinheit 4 erfolgt beim dargestellten Ausführungsbeispiel durch den Traggurt 3. In diesen sind hierzu parallel zueinander in Längsrichtung des Gurtes 3 verlaufend zwei flexible Litzen 31 und 32 eingewebt. Zur Herstellung des elektrischen Kontaktes zwischen dem Litzenpaar 31, 32 und dem Leiterbahnpaar 21, 22 des Fühlers 2 weisen das Gurtende 30 und der Fühler 2 jeweils zwei zueinander kompatible, als Druckknöpfe ausgebildete Kontaktpunkte 33, 34 bzw. 23, 24 auf. Zugleich dienen die Druckknöpfe zur mechanischen Verbindung von Gurt 3 und Fühler 2. Zwecks besserer Übersichtlichkeit der Darstellung sind in Figur 1 der Gurt 3 und der Fühler 2 in voneinander getrenntem Zustand dargestellt. Die zwischen Gurt 3 und Fühler 2 eingezeichneten Bewegungspfeile deuten an, wie die Verbindung zwischen Gurt 3 und Fühler 2 erfolgt. Die Kontaktpunkte 23 und 24 des Fühlers 2 sind jeweils mit einer der Leiterbahnen 21 und 22 verbunden; die Kontaktpunkte 33 und 34 des Gurtes 3 sind entsprechend jeweils mit einer der Litzen 31 und 32 elektrisch leitend verbunden.

Die erwähnte Schalteinheit 4 ist in einem Gehäuse 5 untergebracht, welches auf einer Flachseite des Gurtes 3 befestigt ist. Zur Herstellung des erforderlichen elektrischen Kontaktes zwischen dem Litzenpaar 31 und 32 sowie der Schalteinheit 4 sind durch die gurtzugewandte Seite des Gehäuses 5 zwei Kontaktstifte 41 und 42 geführt, welche bei Verbindung von Gurt 3 und Gehäuse 5 miteinander in den Gurt 3, genauer in die in diesen eingewebten Litzen 31 und 32 einstechbar sind. Hierdurch wird auf einfachste Weise eine Kontaktierung hergestellt.

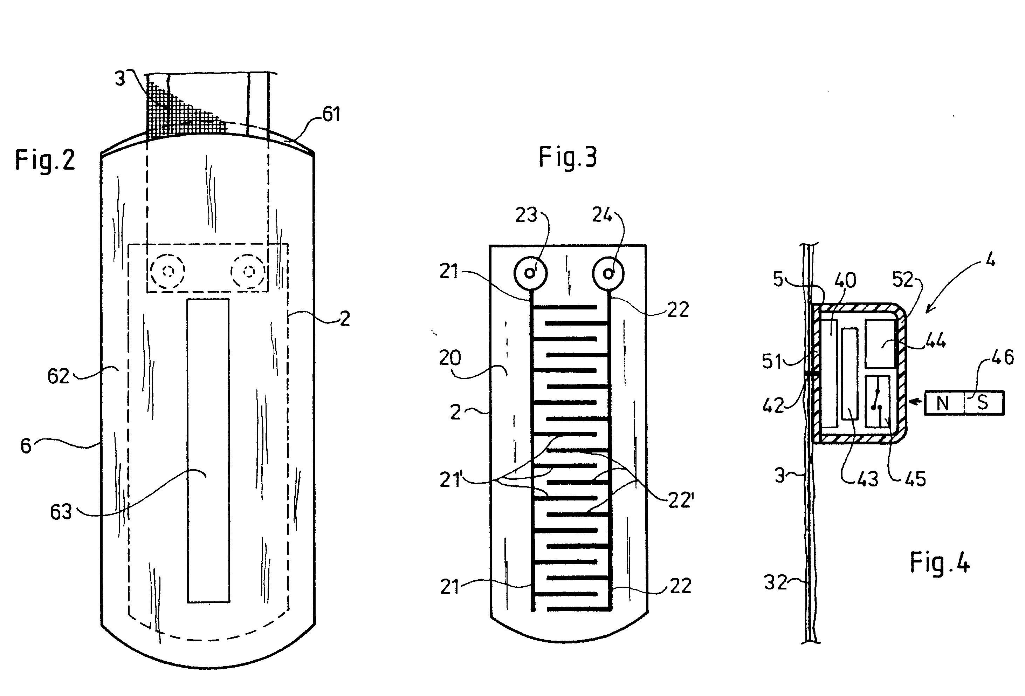

Um allen hygienischen Ansprüchen gerecht zu werden, ist der Fühler 2 bei seinem Einsatz in einer Hülle 6 aus einem feuchtigkeitsdurchlässigen Material untergebracht, wie dies aus der Figur 2 ersichtlich ist. Die Hülle 6 besteht beispielsweise aus saugfähigem Papier oder Zellstoff und weist die Form eines länglichen Etuis auf. Durch einen Einsteckschlitz 61 an der einen Schmalseite der Hülle 6 können der Fühler 2 sowie ein kurzes Stück des Traggurtes 3 in die Hülle 6 eingeführt werden. Zur Fixierung der Hülle 6 mit dem inliegenden Fühler 2 z. B. in einer Unterhose weist eine Flachseite 62 der Hülle 6 im dargestellten Ausführungsbeispiel einen Klebestreifen 63 auf. Wegen ihrer preisgünstigen Herstellbarkeit ist die Hülle 6 als Wegwerfartikel für Einmalgebrauch vorgesehen.

Figur 3 zeigt ein zweites Ausführungsbeispiel eines Fühlers 2 mit einer anderen Anordnung der Leiterbahnen 21 und 22. Während diese beim ersten Ausführungsbeispiel nach Figur 1 im wesentlichen in Längsrichtung des Trägers 20 parallel zueinander verlaufend angeordnet sind, sind bei dem Beispiel des Fühlers 2 nach Figur 3 eine Vielzahl von kammartig ineinander verschachtelten kurzen Leiterbahnstücken 21' und 22' vorhanden, welche jeweils mit einem parallel zum Außenrand des Trägers 20 verlaufenden Leiterbahnabschnitt 21 und 22 verbunden sind. Am einen Ende des Trägers 20 sind die Leiterbahnen 21 und 22 mit ihren zugehörigen kurzen Leiterbahnabschnitten 21' und 22' in der bereits beschriebenen Art und Weise mit Kontaktpunkten 23 bzw. 24 elektrisch leitend verbunden. Außer den in den Figuren 1 und 3 gezeigten Anordnungen der Leiterbahnen sind selbstverständlich auch andere Ausgestaltungen denkbar, die den gleichen Zweck erfüllen, so z.B. auf beiden Seiten des Trägers 20.

Figur 4 schließlich zeigt eine Schalteinheit 4 als Teil der Vorrichtung im Längsschnitt in prinzipieller Darstellung. Die dargestellte Schalteinheit 4 weist mehrere Elemente auf, nämlich einen elektronischen Schaltkreis 40, welcher vorzugsweise als Hybridschaltung ausgeführt ist, eine Batterie 43 zur Energieversorgung der Schalteinheit 4, einen Schallsignalgeber 44 zur Abgabe eines akustischen Warnsignals und ein Reedrelais 45 zur Rücksetzung der Schalteinheit nach einem Ansprechen. Dieses Reedrelais 45 ist mittels eines von Hand in die Nähe gebrachten Permanentmagneten 46 betätigbar.

Der Schaltkreis 40, die Batterie 43, der Signalgeber 44 und das Reedrelais 45 sind in einem Kunststoffgehäuse 5 untergebracht, welches aus einer mit dem Gurt 3 verbundenen Bodenplatte 51 und einem auf diese aufgesetzten Deckel 52 besteht. Wegen der Rücksetzung der Schalteinheit 4 mittels eines Magneten sind im Gehäuse 5 keinerlei Öffnungen erforderlich, so daß die in dessen Innerem angeordneten Elemente vollkommen vor Staub und Feuchtigkeit geschützt sind. Zur Abgabe eines möglichst lauten akustischen Signals bei möglichst geringem Energieverbrauch ist der Schallsignalgeber 44 vorzugsweise mit dem Deckel 52 des Gehäuses 5 verbunden, beispielsweise verklebt, so daß der Deckel 52 zur Schallverstärkung zu Schwingungen angeregt wird.

Weiterhin ist in Figur 4 der eine Stift 42 der beiden Kontaktstifte 41 und 42 erkennbar, welcher von dem Schaltkreis 40 durch den Gehäuseboden 51 in den Gurt 3, genauer in die in diesen eingewebte Litze 32 geführt ist. In gleicher Weise erfolgt die elektrische Verbindung vom Schaltkreis 40 über den anderen Kontaktstift 41 in die andere Litze 31 im Traggurt 3.

Bei Verwendung der beschriebenen Vorrichtung zur Behandlung von an Enuresis leidenden Personen wird der Fühler 2 mit der ihm umgebenden Hülle 6 unmittelbar vor der Harnröhrenöffnung plaziert und fixiert. Der Traggurt 3 wird über die Schulter des Patienten geführt und rückenseitig beispielsweise mittels eines Klett- oder Klammerverschlusses an der Unterhose befestigt. Die Schalteinheit 4 mit ihrem Gehäuse 5 ist vorzugsweise im Schulterbereich mit dem Gurt 3 verbunden, so daß sie in der Nähe des Ohres des Patienten angeordnet ist und in Liegestellung nicht störend in Erscheinung tritt. Bei einem Auftreffen von Feuchtigkeit auf dem Fühler 2 infolge beginnenden, unkontrollierten Harnlassens werden die Leiterbahnen 21 und 22 des Fühlers 2 leitend miteinander verbunden, woraufhin von der Schalteinheit 4 ein akustisches Warnsignal abgegeben wird, welches zum unmittelbaren Aufwachen des Patienten führt. Wegen der hohen Ansprechempfindlichkeit der Vorrichtung wird der Patient wach, bevor die Harnblase entleert ist, so daß eine Wahrnehmung des Harndranggefühles ermöglicht wird. Hierdurch wird mit der Zeit beim Patienten ein Lerneffekt erzielt, der den Gebrauch der Vorrichtung nach einiger Zeit überflüssig macht. Aufgrund der Ausgestaltung der Vorrichtung besitzt diese eine sehr hohe Funktionssicherheit, so daß mit dieser eine optimale, nicht durch technische Fehler verzögerte oder beeinträchtigte Behandlung des Enuresis-Patienten ermöglicht wird. Zudem kann die Schalteinheit der Vorrichtung sehr kompakt und leicht ausgeführt sein, so daß das Tragen der Vorrichtung für den Patienten keine Belastung darstellt.

**Patentansprüche**

1. Vorrichtung zur Erzeugung eines Warnsignals bei unkontrolliertem Harnaustritt, insbesondere zur Behandlung der Enuresis, mit einem im Bereich der Harnröhrenöffnung anzuordnenden Feuchtigkeitsfühlers (2), der auf einem Träger (20) angeordnet ist, wobei der Feuchtigkeitsfühler (2) mit durch eventuell austretenden Urin überbrückbaren unisolierten elektrischen Leiter bahn paar (21, 21', 22, 22') versehen ist, mit einer mit dem Fühler (2) elektrisch verbindbaren, am Körper tragbaren elektrischen oder elektronischen Schalteinheit (4) zur Feststellung eines Auftretens von Feuchtigkeit am Fühler (2), wobei die Schalteinheit (4) mittels eines Magneten (45) über ein einen Teil der Schalteinheit (4) bildendes Reedrelais (45) schaltbar ist, und mit einem von der Schalteinheit (4) im Ansprechfall ansteuerbaren Schallsignalgeber (44), und wobei der Fühler (2) von einer leicht auswechselbaren, für eine einmalige Verwendung vorgesehenen, flüssigkeitsdurchlässigen Hülle (6) umgeben ist, **dadurch gekennzeichnet,** daß die Schalteinheit (4) und damit der Schallsignalgeber (44) nach einem Ansprechen durch Schließen des Reedrelais (45) mit Hilfe einem Magneten abschaltbar ist.

2. Vorrichtung nach Anspruch 1, dadurch gekennzeichnet, daß die Schalteinheit (4) einen als Hybridschaltung ausgebildeten Schaltkreis (40) mit zugeordneter Batterie (43) aufweist, daß der Schaltkreis (40), die Batterie (43), das Reedrelais (45) und der Schallsignalgeber (44) gemeinsam innerhalb eines Gehäuses (5) angeordnet sind und daß das Gehäuse (5) seinerseits mit einem im Anwendungsfall über die Schulter eines Patienten führbares Traggurt (3) im Schulterbereich, d.h. in Ohrnähe des Patienten, verbindbar ist.

**Claims**

1. A device for generating a warning signal in the case of uncontrollable urination, in particular, for the treatment of enuresis, with a moisture detector (2) disposed close to the ureter outlet and attached to a strap, whereby the moisture detector (20) is furnished with an unisolated electric conductor pair (21, 21'; 22, 22') that can be bridged by means of contingently emerging urine, with electrical or electronic switching equipment (4) which can be connected electrically to the detector (2) and can be carried on the body, in order to detect the emergence of moisture at the detector (2), whereby the switching equipment (4) is switchable by means of a magnet (46) via a dry-reed relay (45) which forms part of the switching equipment and a sound signaller (44) triggered in the case of a response, and whereby the detector (2) is enveloped in a sheath permeable to liquids and ependable after a single employment and which can be changed easily,

characterized in that the switching equipment (4) and thus the sound signaller (44) can, after a response through the closing of the dry-reed relay (45), be switched off with the help of a magnet.

2. Device according to claim 1, characterized in that the switching equipment (4) is furnished with a circuit (40) of a hybrid type and the requisite battery (43), in that the circuit (40), the battery (43), the dry-reed relay (45) and the sound signaller (44) are arranged together in a casing (5) and in that this casing (5) can, in its turn, be connected to a carrying strap (3) which, when employed, can be carried over the shoulder of a patient, that is, so that the casing is close to the patient's ear.

**Revendications**

1. Appareil destiné à produire un signal avertisseur en cas d'émission incontrôlée d'urine. A utiliser en particulier dans le traitement de l'énurésie. Comporte un détecteur d'humidité (2) à mettre en place dans la région de l'urètre; ce détecteur est placé sur un support (20). Le détecteur d'humidité (2) est muni d'un couple de pistes conductives (21, 21' 22, 22') électriques, non isolées, court-circuitables lors d'une émission éventuelle d'urine. Comprend aussi une boîte de commutation (4) électrique ou électronique reliable électriquement au détecteur (2) et portable sur le corps, permettant d'établir la présence d'humidité au détecteur (2), la boîte de commutation (4) étant commutable grâce à un aimant (46) par l'intermédiaire d'un relais reed (45) qui forme une partie de la boîte de commutation (4); comporte également un émetteur de signal acoustique (44) commandé en cas de réponse par la boîte de commutation (4), le détecteur (2) étant enveloppé d'une gaine (6) perméable à l'eau prévue pour une seule utilisation et facilement remplacable,
appareil caractérisé par le fait
que la boîte de commutation (4) et avec elle l'émetteur de signal acoustique (44) sont déconnectables après une réponse par fermeture du relais reed (45) à l'aide d'un aimant.

2. Appareil présentant les spécifications décrites en 1 caractérisé par le fait que la boîte de commutation (4) présente un circuit de commutation (40) hybride avec batterie (43), que le circuit de commutation (40), la batterie (43), le relais reed (45) et l'émetteur de signal acoustique (44) sont disposés ensemble à l'intérieur d'un boîtier (5) et que ce boîtier (5) est pour sa part reliable à une bretelle (3) ajustable en cas d'utilisation à l'épaule d'un patient, et se trouve donc dans la région de l'épaule du patient, c'est-à-dire à proximité de son oreille.

Fig. 1

Fig.2

Fig.3

EP 0 270 048 B1

Fig.4